# EUROPEAN PATENT APPLICATION

(11) **EP 3 865 582 A1**
(43) Date of publication of application: **18.08.2021**
(21) Application number: 19856460.1
(22) Date of filing: 13.03.2019
(51) Int. Cl.: C12N 15/90, C12N 5/10

(54) **HAFFT1 CELL**

(30) Priority: 30.09.2018 CN 201811153259
(71) Applicant: Beijing DCTY Biotech Co., Ltd., Beijing 102200 (CN)
(72) Inventor: JIAO, Shunchang, Beijing 100000 (CN); ZHANG, Rong, Beijing 100000 (CN); ZHANG, Tianfu, Beijing 100000 (CN); ZHOU, Zishan, Beijing 100000 (CN); CHEN, Xiaobin, Beijing 100000 (CN); LI, Yingying, Beijing 100000 (CN); PENG, Gang, Beijing 100000 (CN)
(74) Representative: Lang, Christian
(86) International application number: PCT/CN2019/077967
(87) International publication number: WO 2020/062795

(57) **Abstract**

The invention discussed in this paper falls under the biotech field, and it is concerned with a specific specie of HAFFT1 cell and the related preparation procedures. The TCR-T technology is used to modify the cell, and the immunosuppressive target of the modified T cell is knocked out. The specific killing T cells are precisely protected from inhibition in vivo, and the cytotoxicity of T cells to tumor cells is improved. The percentage of specific T cells (TCR+) that can recognize tumor antigens is more than 80% after HAFFT1 modification.

## Description

### Technical FIELD:

The invention discussed in this paper (hereinafter "Invention") falls under the biotech realm, and it is concerned with a specific specie of HAFFT1 cell and the related preparation procedures.

### Background ART:

It has been proven that existing LAK, DC, CIK, DC-CIK cells and associated methods are largely ineffective in the treatment of specific immunotherapy of tumors at present, while the technics relating to NK, CAR-NK, TIL cells have yet to mature, and there are still many shortcomings in the safety and efficiency of CAR-T therapy for treatment of solid tumor in clinical trials.

The existing technology generally produces specific killing by modifying DC cells and presenting T cells by DC. By using viruses as vectors to transfect presenting T cells, some laboratories could induce specific killing of T cells. In our previous study, we have also directly stimulated PBMC with mutant mixed polypeptide to induce T cells. Other laboratories use TCR-T technology to target-present MAGE A3 antigen.

The above therapeutic methods are not mature, especially in the theoretical study of inducing DC cells and DC cells to load tumor antigens in vitro. However, there are still many problems in the process of implementation, such as the lack of clear molecules related to the occurrence and development of tumor cells as induced antigens. Because the tumor antigens are unknown and the immunosuppression of tumor microenvironment is impaired, it is difficult to implement specific immunotherapy based on cells. In addition, although some methods involve T cell priming by antigens in vitro, they are difficult to induce effective T cell killing without co-stimulation and amplification, because these specific cells can't infiltrate into the tumor microenvironment successfully and remain cytotoxicity for a long time. Others can also be presented and co-cultured in vitro, but the target is single (MAGE-3), only effective on individual cancers such as non-small cell lung cancer. Although there are some methods of transfection and presentation using lentivirus as vector, the safety and convenience are not as good as that of polypeptide. The direct stimulation of simple mixed polypeptide is simple and convenient, but its efficiency is low. Secondary stimulation of specific precise polypeptide is not as direct as that of tumor specific antigen transduced by T cell receptor. The existing TCR-T lacks accurate TCR to cover more tumor species in solutions for the treatment of hematological and solid tumors.

The methods discussed above lack the consideration of the self-defense technology of T cells, so that a limited amount of specific T cells is exposed directly to a complex and severe tumor immunosuppressive microenvironment.

### SUMMARY OF THE INVENTION:

This Invention provides a solution to the technical problems discussed in the above by offering a specie of HAFFT1 cell. The cell is stimulated by polypeptides modified with small molecules and modified by TCR-T technology, and the immunosuppressive target of the modified T cell is knocked out. The specific killing T cells are precisely protected from inhibition in vivo, and the cytotoxicity of T cells to tumor cells is improved.

The preparation method of the HAFFT1 cell includes the following key steps:
1) screening out mutation sites by ctDNA exon sequencing using peripheral blood taken from patients or whole exome sequencing using tumor tissue to predict antigenic determinants and synthesize mutant polypeptides modified with small molecules;
2) loading the mutant polypeptides modified with small molecules on immortalized DC prepared from peripheral blood, and co-incubating with PBMC to obtain HAFF cells;
3) stimulating the HAFF cells with the mutant polypeptides modified with small molecules as antigens and screen out precise polypeptides;
4) co-incubating the immortalized DC cells loaded with the precise polypeptides with PBMC to prepare HAFF' cells;
5) screen out the specific T cells which can recognize the precise polypeptides by stimulating the HAFF' cells with the precise polypeptides as antigens, and obtaining high frequency TCR of the specific cells by sequencing, and knocking out the original TCR and expressing the high frequency TCR to construct TCR-T cells; and
6) HAFFT1 cells are obtained by knocking out the immunosuppressive signal molecules in the TCR-T cells.

The specific preparation steps of the HAFFT1 cell are as follows:
1. Whole exome sequencing
   This Invention uses human peripheral blood for ctDNA sequencing or tumor tissue for whole exome sequencing, the sequencing results are then compared with the genomes of normal cells to screen out mutation sites.
   The peripheral blood may be commercial engineering cell lines, for example, H1299, H226, H358, H1563, H2228, A549, Renca, mouse Lewis lung cancer (LLC) cells, CRL-6323 B16F1, CRL-2539 4T1, mouse U14 cervical cancer cells, mouse BV-2 microglioma cells, mouse G422 glioma cells, etc, for whole exon sequencing.
2. Prediction of antigenic determinants
   (1) The mutant amino acid site is used as the target center; 8 amino acids are extended on both sides of the target center so that the polypeptides of the 17 amino acid are then used as the "potential antigenic determinants".
   (2) IC50 of potential antigenic determinants is analyzed by using predictive software, such that the potential antigenic determinants with IC50 < 1000 nM is considered as antigenic determinants.
3. Synthesis and modification of polypeptides
   (1) Synthesis of mutant polypeptides from antigenic determinants.
   (2) The small molecule (H4) and polypeptide antigen (P) are modified by the following links (amino to carboxyl: N-C): H-P, P-H, H-P-H.
4. Immortalized DC loaded with mutant polypeptide
   (1) Dendritic cells in peripheral blood are infected with TAX-GFP lentivirus, and ideal clones are selected as immortalized DC.
   (2) Antigen polypeptides modified with small molecules is then loaded to immortalized DC.
5. Co-incubation of DC loaded with mutant polypeptides and PBMC
   HAFF cell is derived from the co-incubation of mutant polypeptides loaded DC and PBMC.
6. Screening of precise polypeptides
   T cells of HAFF cells are first collected, which are then simulated by the mutant polypeptides modified with small molecules obtained in step 3, followed by the screening of precise polypeptide via the examination of the secretion of IFN-y.
7. The preparation of HAFF' cell with the screened precise polypeptide
   Preparation of precise polypeptide HAFF' cells by repeating steps 3, 4 and 5 with precise polypeptides instead of mutant polypeptides.
8. Determination of high frequency TCR in specific cells and construction of its expression vector
   1) HAFF' cells are first stimulated with small molecule modified precise polypeptide, the stimulated cells are then stained with CD8, CD137 and IFN-γ. CD8+CD137+ or CD8+IFN-γ+T cells are selected. Genomes are extracted and TCR is sequenced and analyzed, and the sequence of high frequency TCR is determined according to the distribution frequency of TCR.
   (2) According to the sequence of high frequency TCR, the primers are designed to amplify TCR gene, and the expression vector of TCR gene is then constructed, and the virus is packaged.
9. Construction of CRISPR vector with knockout of immunosuppressive signaling molecules, and packaging with virus.
10. Construction of CRISPR vector for TCR knockout, and packaging with virus.
11. Modification of TCR

The CD8 + T cells screened out from step 8 are infected by virus obtained in steps 9 and 10 and the original TCR and immunosuppressive signaling molecules are knocked out. Then the TCR expression vector constructed in step 8 is transferred to prepare HAFFT1 cells.

The immunosuppressive signal molecule could be PD-1, Tim-3, LAG3, CTLA-4, BTLA, VISTA, CD160 or 2B4 (CD244), TIGIT.

The beneficial effects of the Invention are as follows:
1. The HAFFT1 cells provided by the Invention use tumor antigen as mutant antigen. The HAFFT1 cells are equipped with strong target specificity which do not miss targets easily unlike the other tissues.
2. The Invention produces a high proportion of specific cells. In general, cells that recognize tumor antigens are less than 0.5% in PBMC. The percentage of specific T cell (TCR+) that can recognize tumor antigens is more than 80% after the HAFFT1 modification.
3. The HAFFT1 cells obtained in this Invention have knocked-out immunosuppressive targets such as PD1, CTLA4, TIM3 and LAG3, resulting in a higher killing frequency and unrestricted killing ability against the tumor.

### BRIEF DESCRIPTION OF DRAWINGS

FIG. 1 is a diagram of microscopic observation of DC morphology.
FIG. 2 is a diagram of efficiency of DC loaded with polypeptides.
FIG. 3 is a diagram of detection of AFF cell typing.
FIG. 4 is a diagram of screening of precision polypeptides.
FIG. 5 is a diagram of flow cytometry to detect the proportion of specific T cells.
FIG. 6 is a diagram of TCR distribution frequency.
FIG. 7 is a diagram of knockout of inhibitory targets.
FIG. 8 is a diagram of knockout efficiency detection of the original TCR.
FIG. 9 is a diagram of expression efficiency of the specific TCR.
FIG. 10 is a diagram of killing efficiency of LDH release detection.
FIG. 11 is a diagram of detection of cytokine IFN-γ release by ELISA.
FIG. 12 is a diagram of improvement of the survival situation of the tumor-bearing mice after applying HAFFT1 cell.

### Implementation details:

The technical solution in the embodiments of the Invention is described clearly and completely in conjunction with the diagrams of the embodiments of the Invention. Obviously, the embodiments described here do not represent the entire embodiments of the Invention. In addition, all other embodiments acquired by other researchers without creative work involved would be deemed as within the scope of protection of the Invention.

The definitions of the professional terms HAFFT1 are given below: wherein,
H: refers to polypeptide antigen modified with small molecular technology.
A: refers to immortalized DC technology.
FF: refers to mixed polypeptide stimulation technology.
T: refers to TCR-T technology.
1: refers to target knockout protection technology.

HAFFT1 cells are the synthesis using all of the above techniques.

### Embodiment 1:

This embodiment uses lung cancer patients as example and offers the preparation method of HAFFT1 cell specific to the example.
1. Whole exome sequencing
   1) The peripheral blood of lung cancer patients is taken for sequencing of ctDNA and HLA typing.
   2) Use software to analyze the sequencing information: the mutation sites are screened by comparing the results of ctDNA sequencing and the genomes of normal cells.
2. Prediction of antigenic determinants
   1) The mutant amino acid site is used as the target center; 8 amino acids are extended on both sides of the target center so that the polypeptides of the 17 amino acid are then used as the "potential antigenic determinants".
   2) IC50 of potential antigenic determinants is analyzed by using predictive software (recommended software: NetMHCpan 3.0, PickPocket, and artificial neural networks (ANN)), such that the potential antigenic determinants with IC50 < 1000 nM is considered as antigenic determinants.
3. Synthesis of polypeptides
   The synthesis of mutant polypeptides from "antigenic determinants" is conducted by the third-party technical service company.
4. Modification of polypeptides by small molecule
   1) The links between small molecules (H4) and polypeptide antigen (P) are as follows (amino to carboxyl: N-C): H-P, P-H, H-P-H.
   2) T cells are cultured with small molecule modified polypeptides H-P or P-H or H-P-H by mixed polypeptides stimulation. The proportion of T cells obtained that are specific to antigen polypeptide is higher than that of crash culture of polypeptide antigen (P) alone.
5. Immortalized DC
   1) 100 ml of peripheral blood is extracted from patients.
   2) PBMC is separated by Ficol density gradient centrifugation.
   3) Dendritic cells are isolated with the Dendritic Cell Isolation Kit of Miltenyi Biotec and suspended in culture medium.
   4) The isolated dendritic cells are infected by TAX-GFP lentivirus and cultured statically in incubator at 37 DEG C for observation.
   5) After clonal growth of cells, clones are selected and cultured on 96 well plates.
   6) Phenotypic analysis using monoclonal antibodies.
   7) Ideal clones are selected as immortalized DC.
6. Mutant polypeptides modified with small molecules are loaded onto immortalized DC.
   1) Preparation of polypeptide solution is as follows: the mutant polypeptides modified by step 4 are used for preparing polypeptide solution as reserve. The final concentration of each polypeptide is 10-100 µg/mL, and the preferred concentration is 50 µg/mL.
   2) The immortalized DC collected by centrifugation is suspended in the prepared polypeptide solution and loaded with polypeptides in the cell culture plate.
   3) The immortalized DC loaded with polypeptides is pulsed for 1-4 hours (4 hours preferably) at 37 DEG C with 5% CO2 as reserve.
7. DC loaded with mutant polypeptides is co-incubated with PBMC.
   1) Pre-paved stimulating factor OKM-25: 40 µL OKM-25 and 4 mL PBS, 2 mL/dish (4.5 cm²) at room temperature for 4 hours, and at 4 DEG C for reserve.
   2) The DC loaded with mutant polypeptides is mixed with PBMC at the ratio of 1:50 to 1:500, and the preferred ratio is 1:100, which is transferred to the cell culture plate or flask pre-paved with OMK25.
   3) The DC loaded with mutant polypeptides is shaken and cultured at 37 DEG C with 5% CO₂ and record the time as day 0.
   4) The co-cultured cells are observed. On the fifth day, according to the density of cells, the co-cultured cells are transferred to a large culture flask. 20 mL of fresh culture medium, OKM-100+12% FBS, is added to the 75 cm² culture flask.
   5) On the 7th day of co-culture, 20 mL of fresh OKM-100+12% FBS is added.
   6) On the 10th day of co-culture, the culture medium is OKM200 + 5% FBS. The cells are transferred to a 175 cm² culture flask from the 75 cm² culture flask. The transfer method is as follows: the cells are blown with 25 mL of culture medium, OKM-200+5% FBS, then transferred into a large flask, and repeated twice. The culture medium, OKM-200+5% FBS, is supplemented to 200 mL.
   7) The cells of HAFF scheme can be obtained after 14-21 days of culture.
8. Polypeptides are used as antigens to directly stimulate T cells to screen precise polypeptides.
   1) The cells obtained from HAFF scheme are collected by centrifugation. T cells are collected by centrifugation at a speed of 1500 rpm for 5 minutes, then 10 mL of PBS suspended cell are added and counted, and T cells are collected by centrifugation at a speed of 1500 rpm for 5 minutes, and re-suspended by 1640 + 10% FBS + 200 U/mL IL2, and the count is adjusted to 1 x 10⁶ cells/mL;
   2) T cells are discharged into 96 well flat plates with a multi-channel pipette, in the amount of 200 µL per well, and the number of T cells was 2 x 10⁵. Then mutant polypeptides modified by small molecules in step 4 are added with a final concentration of 50 µg/mL, and each polypeptide is provided with three wells.
   3) Set up positive control: T cells + 100 ng/mL OKT3; and negative control: 1640 + 10% FBS + 200 U/mL IL2.
   4) After stimulated for 24 hours at 37 DEG C with 5% CO₂, mutant polypeptides are centrifuged at a speed of 1500 rpm for 10 minutes and 140 µL supernatant is transferred to a new 96 well plate.
   5) The 96-well plate is centrifuged at a speed of 1500 rpm for 10 minutes, and the samples are detected by ELISA (or stored at - 80 DEG C).
9. Criteria for the evaluation of precise polypeptide:
   1). if the positive and negative controls are normal, this data is reliable or it needs to be re-tested vice-versa.
   2). if the secretion of IFN-γ in the experimental group is significantly exceeding the negative control group, the polypeptide is an effective precise polypeptide.
10. Preparation of HAFF' cells with the screened precise polypeptide
   1) Preparation of precise polypeptide HAFF' cells as set out in steps 4, 5, 6 and 7.
11. Culture and isolation of specific killer T cells of mutant antigen
   1) The HAFF' cells obtained in step 10 are stimulated with the selected precise polypeptide as antigen directly. After stimulated for 12-72 hours, the obtained HAFF' cells are used as reserve.
   2) The stimulated T cells are stained with CD8, CD137 and IFN-γ and sorted by flow cytometry. CD8 + CD137 + or CD8 + IFN-γ+T cells are selected.
12. Detection of TCR frequency in CD8+T cells and cloning of high frequency TCR
   1) The genomes of the selected cells are extracted immediately.
   2) Genomes are analyzed by TCR sequencing, and the sequence of high frequency TCR is determined according to the distribution frequency of TCR.
   3) The mRNA of PBMC are extracted and reverse transcribed into cDNA. The gene of TCR is amplified by primers designed according to the sequence of high frequency TCR.
   4) Construction of vectors for TCR gene expression and packaging of virus.
13. Construction of CRISPR vector with knockout of immunosuppressive signaling molecules
   1) The immunosuppressive signaling molecule on the surface of PBMC includes: PD-1, Tim-3, LAG3, CTLA-4, BTLA, VISTA, CD160, 2B4 (CD244).
   2) The exons of immunosuppressive signaling molecules are analyzed and the CDS region of the gene mRNA on pubmed are ascertained, the knockout targets of each exon are predicted separately.
   3) The forward and reverse primers needed for the synthesis of sgRNA are designed. After mixing at the ratio of 1:1, the forward and reverse primers is treated at 95 DEG C for 5 ∼ 60 min and then slowly cool down to form the DNA sequence of sgRNA .
   4) The vector of CRISPR lentivirus expression is digested by double restriction enzyme digestion and linked with double-stranded DNA corresponding to sgRNA, and transferred into competent cells for cloning. After 12 hours, the monoclonal is selected for sequencing and the correct clones are retained.
   5) The plasmid vector of CRISPR lentivirus carrying DNA sequence corresponding to sgRNA is extracted and packaged with virus.
14. Construction of CRISPR vector for TCR knockout
   1) The CDS region of the TCR gene mRNA on pubmed are ascertained, the conservative region of TCR is analyzed, and the knockout targets of the conservative region are predicted.
   2) The construction of TCR knockout vectors and viral packaging are completed according to steps 3 to 5 of step 13
15. Construction of TCR-T that knocks out immunosuppressive signaling molecules
   1) PBMC is resuscitated and CD8 + cells are sorted by magnetic beads for reserve.
   2) CD8 + T cells acquired in step 11 are infected with viruses acquired in steps 13 and
14. Meanwhile, the original TCR and immunosuppressive signaling molecules are knockout.
   3) After infection, CD8 + T cells are cultured in the medium for 0 - 5 days, and optimized for 3 days, and then transferred into the constructed TCR expression vector.
   4) Record the time as day 0 after the infected CD8 + T cells are suspended with OKM100 + 12% FBS and placed on the pre-paved stimulating factor OKM-25.
   5) Observe the condition and density of the cells, and the co-cultured cells are transferred to a large culture flask and fresh medium OKM-100+12% FBS is added on the fifth day.
   6) The cells are transferred from 75 cm² flask to 175 cm² flask, and the medium is OKM-200+5% FBS.
   7) When cultured for 14-21 days, TCR-T or HAFFT1 cells, which knocked out immunosuppressive signal molecules, could be harvested.
16. Construction of target cells expressing specific antigens and survival experiment of tumor model.
   1) Lentiviral vectors expressing precision peptides (specific antigens) selected are constructed.
   2) Lentiviral vectors expressing specific antigens are packaged into lentiviral particles to infect HLA-matched tumor cells, and stabilize over-expression of specific antigens, and the expression level and intensity are detected by flow cytometry.
   3) NGS mice are inoculated with tumor cell lines stably overexpressing specific antigen peptide to establish heterotopic tumor-bearing animal models. 5 x 10⁵ tumor cells expressing specific antigens are suspended in 100 µL saline and subcutaneously injected into the right flank and rib of 30 NSG mice, respectively, and the NSG mice are numbered at the same time.
   4) When the tumor grow to about 100-120 mm³, the animal models are randomly divided into three groups according to the size of tumors, 5-6 mice in each group. One group is given placebo saline, the second group is given 1×10⁷ T cells (control group) without genetic manipulation, and the third group was given 1×10⁷ MRFFT1 cells. The injection of cells will take place with an interval of 7 days after the first injection, and each group of mouse need to be injected 3 times. The survival data is recorded continuously for 60 days and survival curve is drawn based on these data.

### Experiment results:

### 1. Prediction of mutation sites and antigenic determinants

Table 1 shows the predicted results of mutation sites and antigenic determinants detected by sequencing.

**Table 1 Prediction of antigenic determinants**

| polypeptide number | antigenic determinants | affinity with HLA (nM) |
|---|---|---|
| 1 | YSFPARVPPPL | 3.36 |
| 2 | FPARVPPPL | 32.6 |
| 3 | YGRHGAAL | 56.14 |
| 4 | SFPARVPPPL | 9.86 |
| 5 | YSFPARVPPPL | 16.93 |
| 6 | IFFGTSQSADM | 35.34 |
| 7 | SGYGRHGAAL | 127.98 |
| 8 | SGYGRHGAAL | 112.49 |
| 9 | YGRHGAAL | 112.49 |
| 10 | EPGSLEKQ | 151.4 |

### 2. Observation of immortalized DC morphology.

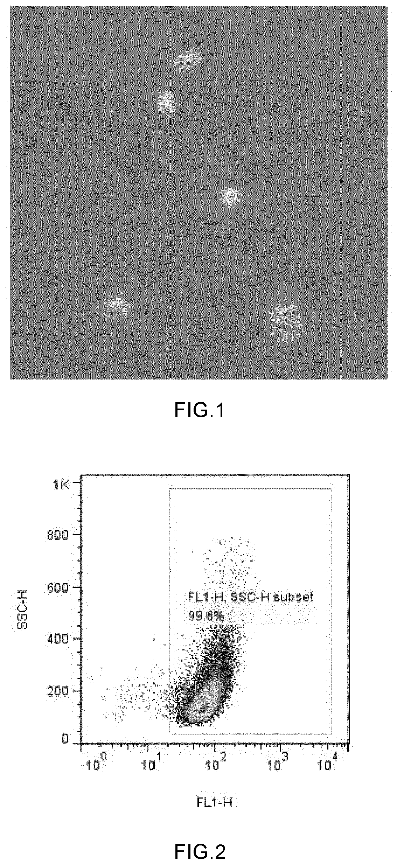

After DC is induced to mature, the morphology of DC is observed under microscope, and obvious dendritic cells could be observed (as shown in FIG. 1)

### 3. Flow cytometry detection of DC antigen loading efficiency.

Predicted mutant antigens are synthesized according to Table 1 and labeled with FITC. After the antigen is loaded with DC, the efficiency of DC-presenting polypeptide antigen is detected by flow cytometry. The result shows that the loading efficiency of DC is 99.6% (as shown in FIG. 2).

### 4. Detection of HAFF cell typing

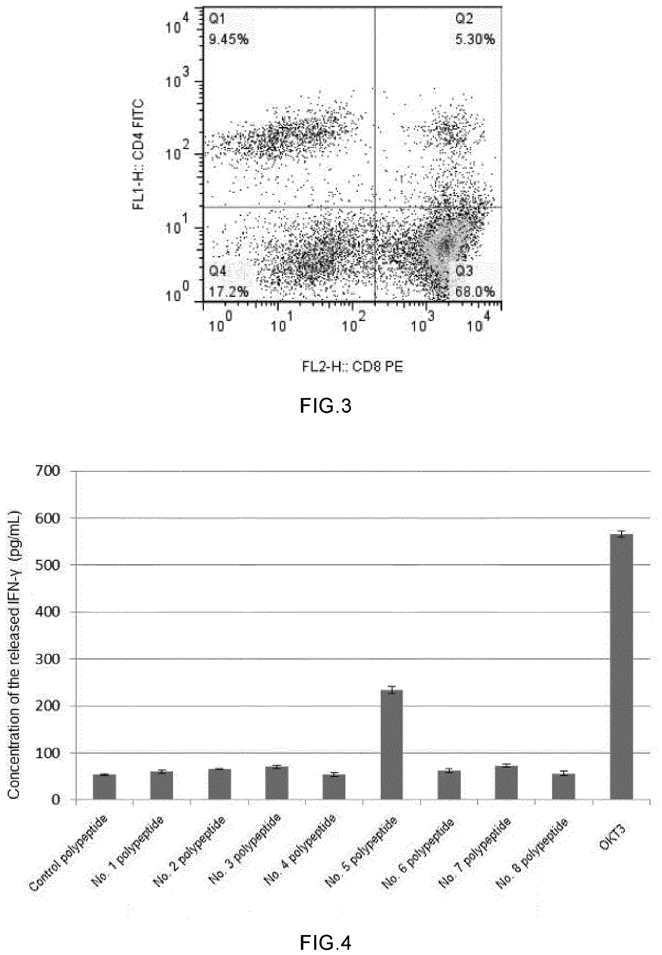

After HAFF cells are cultured, CD4 + cells and CD8 + cells are typed. As shown in FIG. 3, the percentage of CD8 + T cells is 68% and the percentage of CD4 + T cells is 9.45%.

### 5. Screening of precision peptides by HAFF Cells

Ten polypeptides are used to stimulate the cultured T cells respectively. Effective polypeptides are detected by detecting the secretion of IFN-γ. As shown in FIG. 4, the release of IFN-γ secreted by T cells stimulated by No. 5 polypeptide, which is identified as an effective polypeptide, is higher than that of negative control.

### 6. Identification and sorting of T cells specific to precision peptides

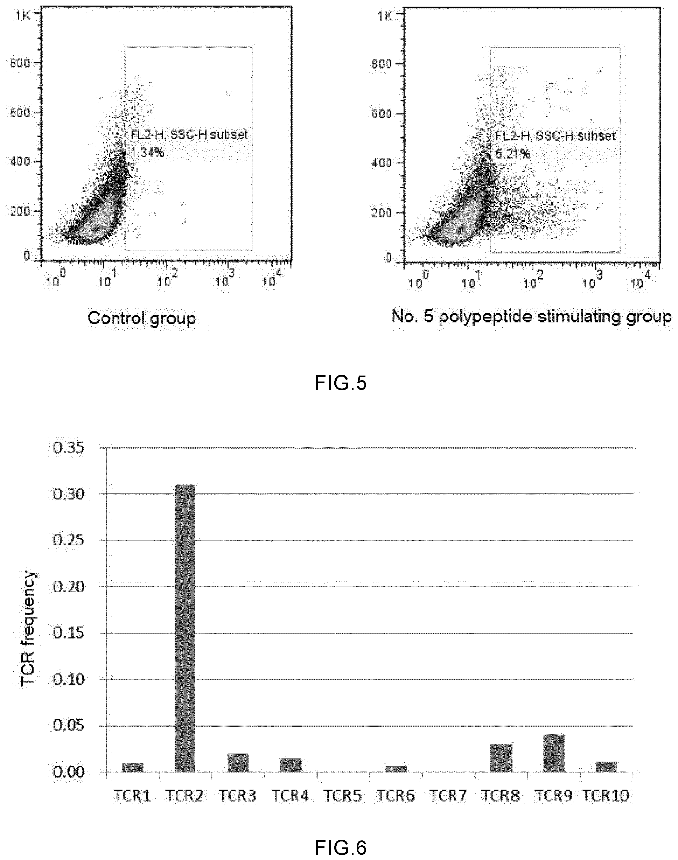

The HAFF' cells are stimulated by the selected No. 5 polypeptide. The proportion of T cells specific to precise polypeptide is detected by flow cytometry. As shown in FIG. 5, specific T cells are found in the box, and the proportion of IFN-γ-releasing cells induced by polypeptide 5 of HAFF' cells are significantly higher than that of non-stimulating cells (control). The results show that specific T cells for effective polypeptide can be obtained by HAFF' scheme. At the same time, CD8 + IFN-γ+T cells (in the box) are sorted by flow cytometry.

### 7. Identification and cloning of high frequency TCR

Genome extraction and sequencing of TCR are performed on the sorted cells. The distribution of TCR is shown in FIG. 6 (the top 10 of high frequency distribution). The frequency of TCR2 is higher. This indicated that TCR is closely related to mutant antigen. TCR is amplified according to TCR sequence and lentivirus expression vector is constructed

**Table 2 Sequence of TCR β-chain CDR3**

| number | 5'-3' DNA sequence Sequence of TCR β-chain CDR3 | CDR3 amino acid sequence |
|---|---|---|
| TCR1 | TGTGCCAGCAGCCCAACGACAGGGGCTGTCTACGAGCAGTACTTC | CASSPTTGAVYEQYF |
| TCR2 | TGTGCCAGCAGCCAAGGGTCCAGCGGGAGAGCCAAAAACATTCAGTACTTC | CASSQGSSGRAKNIQYF |
| TCR3 | TGTGCCAGTAGTATTGGGGCAGGTTTCCATCTCGAGCAGTACTTC | CASSIGAGFHLEQYF |
| TCR4 | TGTGCCAGCAGTTTTCTCAGGGACCCTTACGAGCAGTACTTC | CASSFLRDPYEQYF |
| TCR5 | TGTGCCAGCACCGACCCCAATACCAACGAACTCAATGAGCAGTTCTTC | CASTDPNTNELNEQFF |
| TCR6 | TGCAGCGTTGTGGCACTAGCGGGAGGGCGGACCTCCTACGAGCAGTACTTC | CSVVALAGGRTSYEQYF |
| TCR7 | TGTGCCAGCAGTCCCCTCCACAGGGGTCCACCCCTCCACTTT | CASSPLHRGPPLHF |
| TCR8 | TGTGCCAGCAGTTTGGCGGGAGGGCCTAGCACAGATACGCAGTATTTT | CASSLAGGPSTDTQYF |
| TCR9 | TGTGCCAGCAGTTACTCGTACAGGGGGCCCTACAATGAGCAGTTCTTC | CASSYSYRGPYNEQFF |
| TCR10 | TGTGCCAGCAGTTTATCAGGGGAATTATACGAGCAGTACTTC | CASSLSGELYEQYF |

The known TCR-α:
Amino acid sequence: 
MMKSLRVLLV ILWLQLSWVW SQQKEVEQNS GPLSVPEGAI ASLNCTYSDR
GSQSFFWYRQ YSGKSPELIM FIYSNGDKED GRFTAQLNKA SQYVSLLIRD
SQPSDSATYL CAVNFGGGKL IFGQGTELSV KPN
Base sequence:

ATGATGAAAT CCTTGAGAGT TTTACTAGTG ATCCTGTGGC TTCAGTTGAG
CTGGGTTTGG AGCCAACAGA AGGAGGTGGA GCAGAATTCT GGACCCCTCA
GTGTTCCAGA GGGAGCCATT GCCTCTCTCA ACTGCACTTA CAGTGACCGA
GGTTCCCAGT CCTTCTTCTG GTACAGACAA TATTCTGGGA AAAGCCCTGA
GTTGATAATG TTCATATACT CCAATGGTGA CAAAGAAGAT GGAAGGTTTA
CAGCACAGCT CAATAAAGCC AGCCAGTATG TTTCTCTGCT CATCAGAGAC
TCCCAGCCCA GTGATTCAGC CACCTACCTC TGTGCCGTGA ACTTCGGAGG
AGGAAAGCTT ATCTTCGGAC AGGGAACGGA GTTATCTGTG AAACCCAAT

The known TCR-β:
Amino acid sequence: 
MRIRLLCCVA FSLLWAGPVI AGITQAPTSQ ILAAGRRMTL RCTQDMRHNA
MYWYRQDLGL GLRLIHYSNT AGTTGKGEVP DGYSVSRANT DDFPLTLASA
VPSQTSVYFC ASSLSFGTEA FFGQGTRLTV V
 (The underlined part is the CDR3 sequence, which needs to be substituted).
Substituted TCR-β: 
MRIRLLCCVA FSLLWAGPVI AGITQAPTSQ ILAAGRRMTL RCTQDMRHNA
MYWYRQDLGL GLRLIHYSNT AGTTGKGEVP DGYSVSRANT DDFPLTLASA
VPSQTSVYF CASSQGSSGRAKNIQYF GQGTRLTVV
 (The underlined part is the substituted CDR3 sequence).

### 9. Detection of the knockout efficiency of inhibitory target

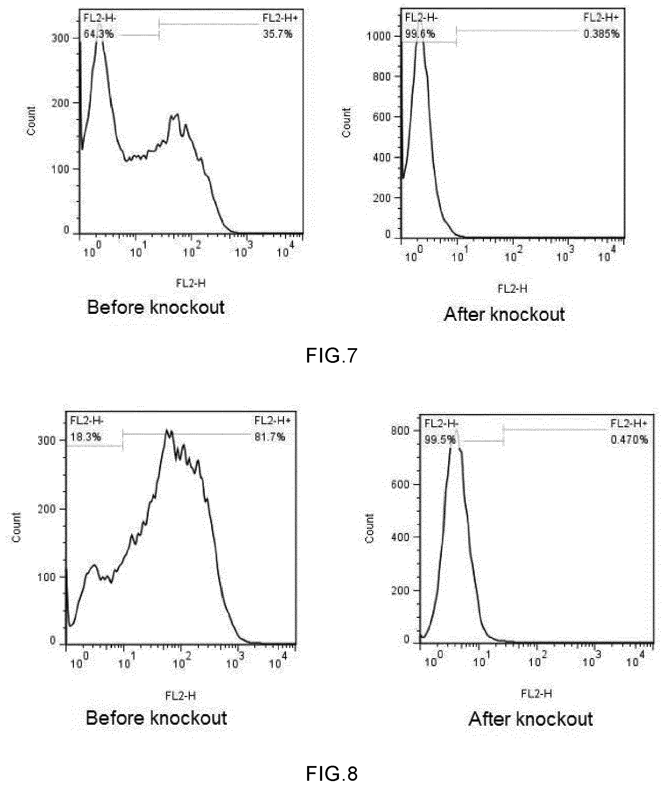

The inhibitory target PD-1 of CD8+T cells is knocked out by CRISPR technology. The sequence of sgRNA is shown in Table 3. The knockout efficiency of the inhibitory target is shown in FIG. 7. The knockout efficiency of sgRNA1 is the highest which can effectively block the expression of the inhibitory signal molecule PD-1. This method may be used to knock out inhibitory signal molecules such as Tim-3, LAG3, CTLA-4, BTLA, VISTA, CD160, 2B4 (CD244).

**Table 3 Inhibitory target sgRNA sequence**

| number | 5'-3' sequence |
|---|---|
| sgRNA 1 | ACAGCGGCACCTACCTCTGTGGG |
| sgRNA2 | ACTTCCACATGAGCGTGGTCAGG |

### 10. Detection of the knockout efficiency of original TCR

The original TCR is knocked out by CRISPR technology. As shown in FIG. 8: It can effectively reduce the expression of the original TCR. At this time, the lentivirus expressing specific TCR can be transfected.

### 11. Detection of specific TCR expression

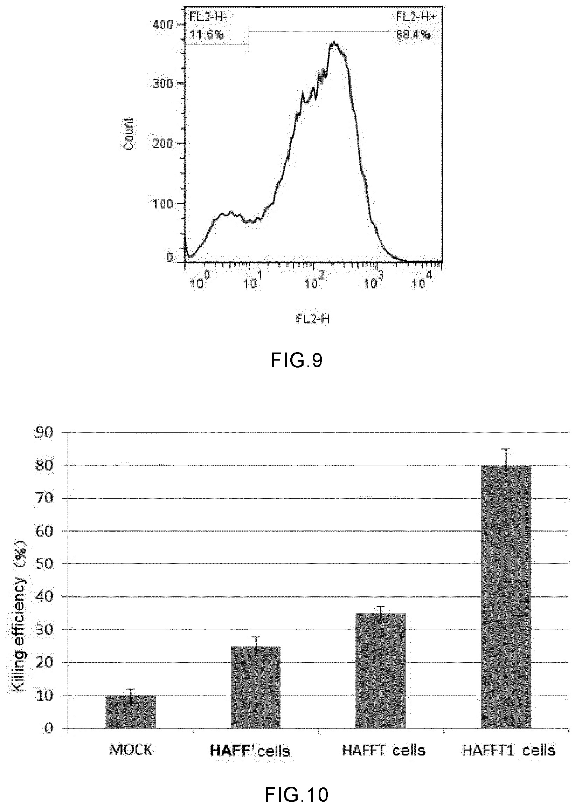

CD8+T cells are transfected with lentiviruses packaged with specific TCR. On the 7th day, the expression efficiency of TCR is detected by flow cytometry. As shown in FIG. 9: the constructed TCR could express normally and the proportion of TCR + cells is 88.4%.

### 12. Killing effect of HAFFT cells on target cells.

The killing efficiency of target cells derived from mutant antigenic determinant is tested by HAFF' cells, HAFFT cells and HAFFT1 cells respectively, and the ratio of effective cells to target cells is set as 40:1. The untreated cells are used as control cells (Mock). The results show that HAFF' cells, HAFFT cells and HAFFT1 cells all have certain killing effects on target cells, and the killing efficiency against tumors is HAFFT1>HAFFT cell > HAFF' cell as shown in FIG. 10. It shows that T cells expressing specific TCR, together with knockout of inhibitory targets, can effectively improve the killing efficiency of tumor cells.

### 13. Detection of cytokines released by HAFFT1 cells

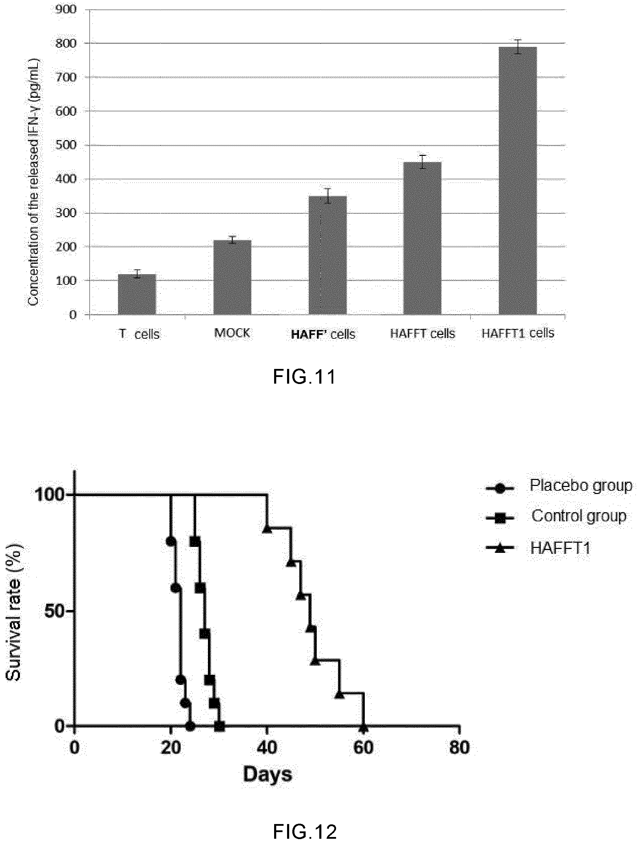

In co-culture of tumor cells and effector cells, because effector cells can recognize mutant antigens of tumor cells, a series of cytokines will be produced. IFN-γ is one of the most important cytokines in anti-tumor effect. FIG. 11 is the detection of IFN-γ released by cells cultured in different ways when co-cultured with tumor cells. The results show that, compared with IFN-γ produced by effector cells themselves (T cells) and effector cells without any modification (MOCK), HAFF' cells, HAFFT cells and HAFFT1 cells all could produce a large number of IFN-γ after co-culture with tumor cells, especially HAFFT cells and HAFFT1 cells. Because of the expression of specific TCR (HAFFT) and the knockout of inhibitory signal (HAFFT1), effector cells can release more IFN-γ. The results are consistent with the killing experiment. It is concluded that T cells expressing specific TCR, combined with closure of inhibitory targets, can enhance the anti-tumor effect more effectively.

### 14. Construction of target cells expressing specific antigens and survival experiment of tumor model

The target cell line expressing specific antigen is successfully constructed and the animal model of tumor bearing is established. The results show that (FIG. 12): HAFFT1 cells can significantly improve the survival of tumor-bearing mice.

### 15. Clinical case

A man: 61 years old.
Disease diagnosis: poorly differentiated adenocarcinoma of both lungs; left pulmonary tuberculosis.
The first course of treatment: HAFFT1 cells are used once a month, and the number of HAFFT1 cells is 1 x 10⁹, 2 times in all;
The second course of treatment: HAFFT1 cells are used once a half month, and the number of HAFFT1 cells is 1 x 10⁹, 2 times in all;

After administration, progression-free survival is 20 months.

**Other cases:**

| patient number | disease diagnosis | CDR3 Sequence of High Frequency TCR | progression-free-survival |
|---|---|---|---|
| 1 | Intrahepatic cholangiocarcinoma | CASSSNDNIPYNEQFF | From August 2016 to the present |
| 2 | ovarian cancer | CASSTERPEQYF | From July 2016 to the present |
| 3 | ovarian cancer | CSAPSGLAGALFYEQYF | From September 2016 to the present |
| 4 | Liver metastasis of gastric adenocarcinoma | CASTSLDYEQYF | From January 2017 to the present |
| 5 | gastric adenocarcinoma | CASSFGRERGFYNEQFF | From May 2017 to the present |
| 6 | lung cancer | CASSPRDRGLTNYGYTF | From May 2017 to the present |
| 7 | esophageal cancer | CASSQEILGGGTDTQYF | From September 2017 to the present |
| 8 | lung adenocarcinoma | CASSSGKWGTEAFF | From January 2018 to the present |
| 9 | lung adenocarcinoma | CASSDWPLNEQFF | From March 2018 to the present |
| 10 | gastric adenocarcinoma | CASSPGTSRDNEQFF | From April 2018 to the present |

| | | | |
|---|---|---|---|
| Note:" to the present" means "one day before the filling date " | | | |

## Claims

1. A specie of HAFFT1 cell as prepared by the following steps:
1) screening out mutation sites by ctDNA exon sequencing using peripheral blood taken from patients or whole exome sequencing using tumor tissue to predict antigenic determinants and synthesize mutant polypeptides modified with small molecules;
2) loading the mutant polypeptides modified with small molecules on immortalized DC prepared from peripheral blood, and co-incubating with PBMC to obtain HAFF cells;
3) stimulating the HAFF cells with the mutant polypeptides modified with small molecules as antigens and screen to obtain precise polypeptides;
4) co-incubating the immortalized DC cells loaded with the precise polypeptides with PBMC to prepare HAFF' cells;
5) screening out the specific T cells which can recognize the precise polypeptides by stimulating the HAFF' cells with the precise polypeptides as antigens, and obtaining high frequency TCR of the specific cells by sequencing, and knocking out the original TCR and expressing the high frequency TCR to construct TCR-T cells; and
6) knocking out the immunosuppressive signal molecules in the TCR-T cells of 5) above to obtain HAFFT1 cells.

2. The HAFFT1 cell according to claim 1, wherein the peripheral blood of patients could be an engineering cell line that is commercially available.

3. The HAFFT1 cell according to claim 1, wherein the prediction of antigenic determinants uses the mutant amino acid site as the target center; 8 amino acids are extended on both sides of the target center so that the amino acid are then used as the "potential antigenic determinants".

4. The HAFFT1 cell according to claim 1, wherein the preferred method for knocking out the TCR gene in peripheral blood cells of patients is CRISPR technology.

5. The HAFFT1 cell according to claim 1, wherein the surface immunosuppressive signal molecules include: PD-1, Tim-3, LAG3, CTLA-4, BTLA, VISTA, CD160, 2B4 (CD244), TIGIT.
